# EUROPEAN PATENT SPECIFICATION

(11) **EP 1 745 148 B1**
(45) Date of publication and mention of the grant of the patent: **09.06.2010**
(21) Application number: 05735444.1
(22) Date of filing: 11.04.2005
(51) Int. Cl.: C12Q 1/68, C12N 15/10, C12N 15/63

(54) **A METHOD FOR IDENTIFYING ACTIVATORS OF GENE TRANSCRIPTION**
VERFAHREN ZUR IDENTIFIZIERUNG VON AKTIVATOREN DER GENTRANSKRIPTION
PROCEDE D'IDENTIFICATION D'ACTIVATEURS DE TRANSCRIPTION GENIQUE

(30) Priority: 19.04.2004 US 563392 P
(43) Date of publication of application: 24.01.2007
(73) Proprietor: PIONEER HI-BRED INTERNATIONAL, INC., Des Moines, Iowa 50309 (US)
(72) Inventor: DUVICK, Jonathan, P., Des Moines, IA 50322 (US); FEDOROVA, Maria, Johnston, IA 50131 (US)
(74) Representative: Bentham, Andrew
(86) International application number: PCT/US2005/012286
(87) International publication number: WO 2005/106037

(56) References cited:
- WO-A-00/75362
- WO-A-96/40892
- WO-A-99/55886
- US-A1- 2002 170 094
- US-B1- 6 709 865
- BRUCE W ET AL: "EXPRESSION PROFILING OF THE MAIZE FLAVONOID PATHWAY GENES CONTROLLED BY ESTRADIOL-INDUCIBLE TRANSCRIPTION FACTORS CRC AND P" PLANT CELL, AMERICAN SOCIETY OF PLANT PHYSIOLOGISTS, ROCKVILLE, MD, US, vol. 12, no. 1, January 2000 (2000-01), pages 65-79, XP000882398 ISSN: 1040-4651 cited in the application
- GOFF S A ET AL: "TRANSACTIVATION OF ANTHOCYANIN BIOSYNTHETIC GENES FOLLOWING TRANSFER OF B REGULATORY GENES INTO MAIZE TISSUES" EMBO (EUROPEAN MOLECULAR BIOLOGY ORGANIZATION) JOURNAL, vol. 9, no. 8, 1990, pages 2517-2522, XP002339327 ISSN: 0261-4189
- CAO M ET AL: "Defining the Bacillus subtilis sigma<W> regulon: a comparative analysis of promoter consensus search, run-off transcription/macroarray analysis (ROMA), and transcriptional profiling approaches" JOURNAL OF MOLECULAR BIOLOGY, LONDON, GB, vol. 316, no. 3, 22 February 2002 (2002-02-22), pages 443-457, XP004470936 ISSN: 0022-2836

## Description

### FIELD OF THE INVENTION

The present invention relates to the field of molecular biology, more particularly to the identification of nucleotide sequences that modulate transcriptional regulatory regions.

### BACKGROUND OF THE INVENTION

Regulation of gene expression is fundamental to some of the most commercially, agriculturally, and medically important biological processes, and much of this regulation occurs at the level of transcription initiation. The expression of many genes is mediated by modulators of transcriptional regulatory regions (e.g., promoters) that increase or decrease gene transcription. Moreover, modulation of gene transcription can have serious implications on cell growth and differentiation, plant pathogen resistance, metabolic pathways, and apoptosis. Therefore, methods for identifying nucleotide sequences that modulate the activity of transcriptional regulatory regions involved in regulation of important biological pathways, such as plant pathogenesis response or mammalian tumor progression, are desired.

Currently available methods do not permit the rapid screening of a large pool of nucleotide sequences to identify sequences that modulate transcriptional regulatory regions. Many of the available methods are cumbersome and require the use of multi-well plates, cell sorters, or custom signature tags to screen a library of nucleotide sequences for potential modulators of gene transcription. Other methods require the expression of multiple polynucleotide constructs and the use of reporter genes to monitor transcriptional activity. Thus, new strategies for rapidly identifying nucleotide sequences that modulate transcriptional regulatory regions are needed. Such methods should permit efficient screening of a large number of nucleotide sequences and permit the identification of sequences that can regulate important physiological pathways by increasing or decreasing the activity of a transcriptional regulatory region.

WO 96/40892 is concerned with tetracycline regulated transcriptional modulators with altered DNA binding specificities. Goff et al (1990) EMBO, 9(8): 2517-2522 is concerned with transactivation of anthocyanin biosynthetic genes tissues. US 6,709,865 is concerned with sunflower lox polypeptides and related compositions. Cao et al (2002) J. Mol. Biol., 316(3) 443-456 is concerned with defining the *Bacillus subtilis* sigma <W> regulon. WO 00/75362 is concerned with a method for identifying novel trancriptional regulatory proteins.

### BRIEF SUMMARY OF THE INVENTION

The present invention provides methods and compositions for the identification of nucleotide sequences that encode peptides, polypeptides, or proteins that modulate the activity of a transcriptional regulatory region. The methods of the invention permit the rapid screening of a large number of candidate nucleotide sequences for potential modulators of transcription. The methods of the invention identify nucleotide sequences that directly modulate transcription by interacting with the transcriptional regulatory region. Alternatively, the methods of the invention also allow for the identification of nucleotide sequences that encode peptides, polypeptides, or proteins that indirectly modulate transcription by influencing proteins or other molecules that interact with the transcriptional regulatory region. In some embodiments, the transcriptional regulatory region is specific to a physiological pathway of interest. Potential physiological pathways of interest include, but are not limited to, pathways involved in metabolism, pathogen resistance, tissue development, and apoptosis.

In one aspect of the invention, the methods for identifying nucleotide sequences that encode peptides, polypeptides, or proteins that modulate a transcriptional regulatory region comprise providing a population of polynucleotide constructs. Each polynucleotide construct in the population comprises a common transcriptional regulatory region operably linked to a candidate nucleotide sequence. Candidate nucleotide sequences may be obtained from any organism and from any complex population of molecules, for example, a cDNA or genomic library.

Transcriptional regulatory regions of the invention may comprise, for example, promoters and/or enhancers. In some aspects of the invention, the transcriptional regulatory region regulates a physiological pathway of interest, such as, for example, tissue development, apoptosis, pathogen resistance, or a metabolic pathway. In a particular embodiment, the transcriptional regulatory region comprises an inducible promoter, such as the maize bronze-1 promoter. Each polynucleotide construct may further comprise an intron.

The population of polynucleotide constructs of the invention is introduced into a population of host cells, and each candidate nucleotide sequence is monitored for changes in expression levels. In the methods of the invention, the candidate nucleotide sequence serves as both a potential modulator of transcription and as a reporter for levels of transcriptional activity. That is, a candidate sequence that encodes a peptide, polypeptide, or protein that increases the activity of the transcriptional regulatory region also stimulates its own expression. Alternatively, a candidate sequence that decreases the activity of the transcriptional regulatory to which it is operably linked decreases its own further expression.

The populations of polynucleotide constructs of the invention may be introduced into host cells from any organism of interest, including mammalian and plant cells. Methods for screening for a modulation in the expression level of each candidate sequence include, but are not limited to, techniques that employ nucleic acid hybridization to a complementary DNA microarray, as described herein. Expression levels of each candidate nucleotide sequence are compared at two or more time points to identify sequences that increase or decrease activity of the transcriptional regulatory region, thereby identifying modulators of transcription.

In another aspect, the invention is directed to a method for identifying nucleotide sequences that encode polypeptides that modulate the activity of a transcriptional regulatory region, wherein multiple populations of polynucleotide constructs comprising different transcriptional regulatory regions are used simultaneously. This embodiment comprises introducing at least a first and a second population of polynucleotide constructs into a population of host cells. The polynucleotide constructs of each population comprise a transcriptional regulatory region operably linked to a candidate nucleotide sequence and further comprises a 5 ' UTR sequence. The transcriptional regulatory region and 5' UTR sequence of each population is unique to that particular population of polynucleotide constructs. The expression levels of each candidate sequence are screened as described above, and a sequence that exhibits significant increases or decreases in expression level is further analyzed to determine which transcriptional regulatory region the identified sequence modulates. In one embodiment, methods for making this determination comprise performing RT-PCR on host cell RNA using primers specific to the identified nucleotide sequence and a 5' UTR sequence unique to a particular population of polynucleotide constructs.

Accordingly, the present invention provides a method for identifying a nucleotide sequence that encodes a polypeptide that modulates the activity of a transcriptional regulatory region, said method comprising:
(a) providing a population of polynucleotide constructs, wherein each of said polynucleotide constructs in said population comprises the transcriptional regulatory region operably linked to a candidate nucleotide sequence;
(b) introducing said population of polynucleotide constructs into a population of host cells; and
(c) screening for a modulation in the expression level of each candidate nucleotide sequence, and thereby identifying a nucleotide sequence that encodes a polypeptide that modulates the activity of said transcriptional regulatory region.

The present invention additionally provides a method for identifying a nucleotide sequence that encodes a polypeptide that modulates the activity of a transcriptional regulatory region, said method comprising:
(a) providing at least a first and a second population of polynucleotide constructs,
   (i) wherein each of said polynucleotide constructs in said first population comprises a first common transcriptional regulatory region operably linked to a candidate nucleotide sequence, and wherein each of said polynucleotide constructs in said first population further comprises a first common 5' UTR sequence;
   (ii) wherein each of said polynucleotide constructs in said second population comprises a second common transcriptional regulatory region operably linked to a candidate nucleotide sequence, and wherein each of said polynucleotide constructs in said second population further comprises a second common 5' UTR sequence;
   (iii) wherein said first and said second transcriptional regulatory regions are non-identical; and,
   (iv) wherein said first and said second 5' UTR sequences are non-identical;
(b) introducing said populations of polynucleotide constructs into a population of host cells;
(c) screening for a modulation in the expression level of each candidate nucleotide sequence, and thereby identifying a nucleotide sequence that encodes a polypeptide that modulates the activity of said first or said second transcriptional regulatory region; and,
(d) determining if the identified nucleotide sequence encodes a polypeptide that modulates the activity of said first or said second transcriptional regulatory region.

Compositions of the invention include libraries of polynucleotide constructs. Isolated libraries comprising a population of polynucleotide constructs are provided. In one embodiment, each polynucleotide construct in the library comprises a common transcriptional regulatory region operably linked to a candidate nucleotide sequence. The polynucleotide constructs of the invention may further comprise an intron. In another embodiment, each polynucleotide construct further comprises a common 5' UTR sequence. In some embodiments, the kits further comprise a complementary DNA microarray. Any kit of the invention may further comprise instructions for use.

The methods and compositions of the invention may be used in any system for which suitable culture and transformation methods exist or are developed as the art advances. Examples of suitable host cells include, but are not limited to, plant cells (dicotyledonous and monocotyledonous), animal cells, fungal cells, and bacterial cells. The host cells of the invention may survive in culture or in whole organism systems. In one embodiment of the invention the plant cells are selected from the group consisting of maize, wheat, sorghum, rice, barley, soybean, alfalfa, sunflower, *Brassica,* and tomato.

### DETAILED DESCRIPTION OF THE INVENTION

The present invention provides methods and compositions for the identification of nucleotide sequences that encode peptides, polypeptides, or proteins that directly or indirectly modulate the activity of a transcriptional regulatory region. The methods of the invention permit the rapid screening of a large number of candidate nucleotide sequences for potential modulators of transcription. The invention further provides methods for identifying nucleotide sequences that encode peptides, polypeptides, or proteins that modulate physiological pathways of interest. Compositions of the invention include libraries of polynucleotide constructs and kits that can be used to practice the methods of the invention.

The methods of the invention are directed to identifying nucleotide sequences that encode peptides, polypeptides, or proteins that modulate the activity of a transcriptional regulatory region. The methods of the invention allow for the screening of a large number of candidate nucleotide sequences in order to identify nucleotide sequences that encode peptides, polypeptides, or proteins that increase or decrease the activity of a transcriptional regulatory region. In one embodiment, the method for identifying such a nucleotide sequence comprises introducing a population of polynucleotide constructs comprising a transcriptional regulatory region operably linked to a candidate nucleotide sequence into a population of host cells and screening for a modulation in the expression level of each candidate nucleotide sequence. While the invention is not bound by any theory of operation, because the candidate nucleotide sequences are operably linked to the transcriptional regulatory region, the candidate sequences should function as both potential modulators of transcription and as reporter sequences for monitoring the activity of the operably linked transcriptional regulatory region. Thus, direct or indirect activation of the transcriptional regulatory region by a peptide, polypeptide, or protein encoded by a candidate nucleotide sequence should increase expression of that particular nucleotide sequence. Likewise, if a candidate nucleotide sequence encodes a peptide, polypeptide, or protein that decreases the activity of the transcriptional regulatory region, subsequent expression of that sequence should be reduced.

The terminology "modulates the activity" of a transcriptional regulatory region is intended to mean an increase or decrease in the efficacy or efficiency of a transcriptional regulatory region as measured by a 0.5%, 1%, 5%, 10%, 20%, 30%, 40%, 50%, 60%, 70%, 80%, 90%, or 100% change in transcript levels of a candidate nucleotide sequence controlled by the transcriptional regulatory region. In one embodiment, modulation of the activity of a transcriptional regulatory region is assessed by comparing the expression level of a candidate nucleotide sequence at two or more time points.

As used herein, "modulator of transcription" refers to a nucleotide sequence that encodes a peptide, polypeptide, or protein that increases or decreases the activity of a transcriptional regulatory region. An "activator" or "inducer" of transcription is intended to mean a nucleotide sequence that increases the activity of a transcriptional regulatory region.

The terminology "directly" modulates the activity of a transcriptional regulatory region is intended to mean that the candidate nucleotide sequence encodes a peptide, polypeptide, or protein that binds the transcriptional regulatory region, and thereby modulates the activity of the transcriptional regulatory region. A peptide, polypeptide, or protein that binds the transcriptional regulatory region may increase or decrease the activity of the transcriptional regulatory region.

The terminology "indirectly" modulates the activity of a transcriptional regulatory region is intended to mean that the candidate nucleotide sequence encodes a peptide, polypeptide, or protein that interacts with a cellular component such as a peptide, polypeptide, protein, RNA, or small molecule that modulates the activity of the transcriptional regulatory region. The peptide, polypeptide, or protein encoded by the candidate nucleotide sequence may produce, alter, or modify a cellular component that modulates the activity of the transcriptional regulatory region. The peptide, polypeptide, or protein encoded by the candidate sequence of interest may interact with a cellular component of a signal cascade that results in modulation of the activity of the transcriptional regulatory region. The peptide, polypeptide, or protein encoded by the candidate nucleotide sequence may interact with a cellular component that interacts with a component of a sequence of cellular components that results in modulation of the activity of the transcriptional regulatory region. The peptide, polypeptide, or protein encoded by the nucleotide sequence of interest may interact with a component of a physiological pathway, including but not limited to, a component 1, 2, 3, 4, 5, 6, 7, 8, 9, 10, 11, 12, 15, or 20 molecules removed from the cellular component that directly interacts with the transcriptional regulatory region.

A "transcriptional regulatory region" is intended to mean any nucleotide sequence that directs transcription of a nucleic acid. Transcriptional regulatory regions include, for example, promoters or enhancer regions. Transcriptional regulatory regions may be derived from any source, including, but not limited to, prokaryotic and eukaryotic cells. As used herein, "promoter" includes reference to a region of DNA upstream from the start of transcription that is involved in the recognition and binding ofRNA polymerase and other proteins needed to initiate transcription. "Operably linked" is intended to mean a functional linkage between sequences, wherein transcription may be initiated in one sequence and transcription continues throughout the operably linked sequences. Generally, operably linked means that the nucleic acid sequences being linked are contiguous and, where necessary to join two protein coding regions, contiguous and in the same frame.

As used herein, "population" refers to a group or collection. A "candidate nucleotide sequence" or "candidate sequence" is intended to mean a nucleotide sequence that is to be tested to determine if it encodes a peptide, polypeptide, or protein that modulates the activity of a transcriptional regulatory region. Candidate nucleotide sequences may be obtained from cDNA, genomic DNA, *in vitro* mutagenized or recombined DNA or any complex population of molecules. The candidate nucleotide sequences of the invention include open reading frames or fragments of open reading frames that encode polypeptides or polypeptide fragments. In a particular embodiment, the candidate nucleotide sequences are maize cDNAs. The library of candidate nucleotide sequences is generated by methods particular to the library type (cDNA, genomic DNA, or *in vitro* mutagenized molecules) and the source (plant, mammalian, animal, fungal, or bacterial cells, or *in vitro* reaction). Candidate nucleotide sequences of the invention may be obtained from any organism of interest, in particular, plants. Mutagenized nucleic acid sequences may be obtained by any method known to one of skill in the art, including but not limited to, site directed mutagenesis, UV irradiation, chemical treatment, passage through mutagenic strains, and exposure to modifying agents. Methods of library construction are well known to one of skill in the art. See, for example, Ausubel et al. (1995) Current Protocols in Molecular Biology (Greene Publishing and Wiley-Interscience, New York); Sambrook et al. (1989) Molecular Cloning: A Laboratory Manual (2d ed., Cold Spring Harbor Laboratory Press, Plainview, New York).

A population of polynucleotide constructs for use in the methods of the invention is generated by cloning a library of candidate nucleotide sequences into a population of polynucleotide constructs comprising a transcriptional regulatory region. The polynucleotide constructs are prepared such that the transcriptional regulatory region is operably linked to a candidate nucleotide sequence. The polynucleotide constructs of the invention may further comprise an intron. In a particular embodiment, the intron is located 5'to the candidate nucleotide sequence. Once this population of polynucleotide constructs has been generated, the sequences are transferred by methods known in the art to a vector appropriate for delivery of polynucleotides to a particular host cell. Vectors for delivery of polynucleotides to a variety of host cells are well known in the art.

Construction of a library of candidate nucleotide sequences and polynucleotide constructs of the invention will typically be optimized to minimize the frequency of multiple occurrences of the same candidate nucleotide sequences in a population. Each candidate nucleotide sequence will occur rarely in the total population of polynucleotide constructs, more optimally, each candidate nucleotide sequence will occur only once in the population. It is not necessary that the population include all possible candidate nucleotide sequences from a library of candidate sequences. Moreover, the population of polynucleotide constructs may comprise more than one polynucleotide construct having the same candidate nucleotide sequence.

In some embodiments, a polynucleotide construct comprising a transcriptional regulatory region operably linked to a candidate nucleotide sequence is contained on a plasmid, for example, the *Agrobacterium* T-DNA plasmid. Typical plasmids of interest include vectors having defined cloning sites, origins of replication, and selectable markers. The plasmid may further include transcription and translation initiation sequences and transcription and translation terminators. Plasmids can also include generic expression cassettes containing at least one independent terminator sequence, sequences permitting replication of the cassette in eukaryotes, or prokaryotes, or both, (e.g., shuttle vectors) and selection markers for both prokaryotic and eukaryotic systems. Vectors are suitable for replication and integration in prokaryotes, eukaryotes, or optimally both. For general descriptions of cloning, packaging, and expression systems and methods, see Giliman and Smith (1979) Gene 8:81-97; Roberts et al. (1987) Nature 328:731-734; Berger and Kimmel (1989) Guide to Molecular Cloning Techniques, Methods in Enzymology, Vol. 152 (Academic Press, Inc., San Diego, California); Sambrook et al. (1989) Molecular Cloning: A Laboratory Manual, Vols. 1-3 (2d ed; Cold Spring Harbor Laboratory Press, Plainview, New York); and Ausubel et al., eds. (1994) Current Protocols in Molecular Biology, Current Protocols (Greene Publishing Associates, Inc., and John Wiley & Sons, Inc., New York; 1994 Supplement.

A population of polynucleotide constructs of known complexity is introduced into a population of host cells. The complexity of the population of polynucleotide constructs need only be sufficient to allow for the identification of nucleotide sequences that encode peptides, polypeptides, or proteins that modulate the activity of the transcriptional regulatory region. One of skill in the art will be able to readily determine the appropriate level of complexity necessary to accomplish the purposes of the invention. Each polynucleotide construct in a population comprises a common transcriptional regulatory region. A "common transcriptional regulatory region" is intended to mean that each polynucleotide construct in a particular population comprises the same transcriptional regulatory region; i.e., identical in sequence. After incorporating the population of polynucleotide constructs into host cells, the cells must be cultured for a sufficient duration to allow for expression from the polynucleotide constructs. The appropriate length of time may be determined easily by one of skill in the art using routine techniques. The expression level of each candidate nucleotide sequence is then monitored for an alteration in its expression.

As used herein, "screening for a modulation in the expression level" of a candidate nucleotide sequence encompasses any method for detection and discrimination of alterations in the expression of nucleotide sequences. In one embodiment, screening for changes in expression levels employs a complementary DNA microarray. A "complementary DNA microarray" is intended to mean a solid support or "chip" comprising nucleic acid molecules attached to the solid support at known locations or "addresses." The arrayed nucleic acid molecules are complementary to the candidate nucleotide sequences of the invention, and the location of each nucleic acid on the chip is known. These DNA chips or microarrays, have been generally described in the art, for example, in U.S. Patent. Nos. 5,143,854, 5,445,934, 5,744,305, 5,677,195, 6,040,193, 5,424,186, 6,329,143, and 6,309,831 and Fodor et al. (1991) Science 251:767-77. These arrays may be produced using mechanical synthesis methods or light-directed synthesis methods that incorporate a combination of photolithographic methods and solid phase synthesis methods.

In one screening approach, the population of host cells comprising the polynucleotide constructs of the invention are harvested en masse at two or more time points designed to capture earlier and later periods of expression. Total RNA is extracted, and a cDNA pool is generated by methods that preferentially amplify mRNA derived from the population of polynucleotide constructs. The cDNA pool is converted to labeled (e.g., biotinylated) polynucleotides (e.g., cRNA or cDNA) and hybridized to the complementary DNA microarray described above. Methods for generating labeled polynucleotides and for hybridizing them to DNA microarrays are well known in the art. See, for example, U.S. Patent Application Publication No. 20020144307; Ausubel et al., eds. (1994) Current Protocols in Molecular Biology, Current Protocols (Greene Publishing Associates, Inc., and John Wiley & Sons, Inc., New York; 1994 Supplement). Hybridization intensities for each nucleic acid molecule on the chip are detected by laser scanning and converted to a quantitative value representing the expression level of each candidate nucleotide sequence. Expression levels of each candidate sequence at two or more time points are compared. Sequences that display significant increases or decreases in expression levels between earlier and later time points directly or indirectly modulate the activity of the transcriptional regulatory region.

The methods of the invention allow for the identification of nucleotide sequences that encode peptides, polypeptides, or proteins that increase the activity of a transcriptional regulatory region, i.e., activators of transcription. In some aspects of the invention, the transcriptional regulatory region comprises an inducible promoter, for example, the maize bronze-1 promoter (see, for example, Roth et al. (1991) Plant Cell 3:317-325*;* Furtek et al. (1988) Plant Mol. Biol, 11: 473-481; Ralston et al. (1988) Genetics 119: 185-197. An "inducible promoter" is intended to mean a promoter that has little or no background activity. "Background activity" is intended to mean the level of transcription driven by the transcriptional regulatory region in the absence of an activator or inducer of transcription. In some embodiments, polynucleotide constructs of the invention comprise an inducible promoter operably linked to a candidate nucleotide sequence. These polynucleotide constructs may further comprise an intron, wherein the intron increases the background expression of the candidate nucleotide sequence operably linked to the inducible promoter. Such introns preserve the promoter's sensitivity to stimulation by activators of transcription. As used herein, "background expression" refers to the level of expression of the candidate nucleotide sequence in the absence of stimulation of the operably linked transcriptional regulatory region by a transcriptional activator. While not intending to be limited to any one mechanism, in some embodiments the intron increases background expression of the candidate nucleotide sequence by functioning as a weak promoter. See, for example, Salgueiro et al. (2000) Plant Mol. Biol. 42:615-622. In other embodiments, the intron increases the background activity of the transcriptional regulatory region, thereby resulting in increased expression of the operably linked candidate nucleotide sequence. Appropriate introns are well known in the art and include, but are not limited to, those disclosed in Xu et al. (1993) Plant Mol. Biol. 22:573-588; Chaubet-Gigot et al. (2001) Plant Mol. Biol. 45: 17-30; Salgueiro *et al., supra;* and Kohler et al. (1996) Mol. Gen. Genet. 251:252-258. In some embodiments, the intron is located 5'to the candidate nucleotide sequence and is fused to the inducible promoter. In a particular embodiment, the intron is a maize ubiquitin 1 intron (e.g., maize Ubil-int1). See, for example, Liu et al. (1995) Biochem. Cell Biol. 73:19-30.

While the invention does not depend on a particular mechanism, the presence of an intron in the polynucleotide constructs comprising an inducible promoter operably linked to a candidate nucleotide sequence may initially result in the production of a small amount of transcript of the candidate nucleotide sequence. If the candidate nucleotide sequence in turn encodes a peptide, polypeptide, or protein that directly or indirectly increases the activity of the inducible promoter, a positive feedback loop may result, thereby leading to significant increases in expression of the candidate nucleotide sequence. That is, the candidate nucleotide sequence, by activating the promoter to which it is operably linked, actually stimulates its own further expression.

The invention further comprises a method for identifying nucleotide sequences that encodes a peptide, polypeptide, or protein that modulates the activity of a transcriptional regulatory region, wherein multiple populations of polynucleotide constructs comprising different transcriptional regulatory regions are used simultaneously. One aspect of this method comprises providing at least a first and a second population of polynucleotide constructs. Each polynucleotide construct in the first population comprises a first common transcriptional regulatory region operably linked to a candidate nucleotide sequence and further comprises a first common 5' UTR sequence. The polynucleotide constructs in the second population comprise a second common transcriptional regulatory region operably linked to a candidate nucleotide sequence and further comprises a second common 5' UTR sequence. The terminology "5' UTR sequence" or "5' untranslated region-(UTR)" refers to a sequence at the 5' end of a gene that is not translated into the protein. "Common" is intended to mean that the transcriptional regulatory region of each polynucleotide construct within a population is identical in sequence; similarly, the 5' UTR sequence of each polynucleotide construct within a population is identical in sequence. While common (i.e., identical) transcriptional regulatory regions and 5' UTR sequences are utilized within any given population of polynucleotide constructs, each of these elements are non-identical between populations of polynucleotide constructs. "Non-identical" is intended to mean that the transcriptional regulatory region of the polynucleotide constructs in any given population of polynucleotide constructs has a sequence that is unique to that population; similarly, the 5'UTR region of the polynucleotide constructs in any given population of polynucleotide constructs has a sequence that is unique to that population. Thus, where a first and a second population of polynucleotide constructs is utilized in accordance with this method of the invention as noted above, the first and second transcriptional regions are non-identical (i.e., unique), and the first and second 5' UTR sequences are non-identical (i.e., unique). In other embodiments, three, four, five, six, or more populations of polynucleotide constructs are used, wherein each population comprises a transcriptional regulatory region and a 5' UTR sequence that are unique to that population of polynucleotide constructs. These populations of polynucleotide constructs are introduced into host cells, and the expression levels of the candidate nucleotide sequences are monitored, as described herein above.

Modulations in the expression level of each candidate nucleotide sequence between at least one earlier and one later time point are identified by, for example, use of a complementary DNA microarray, as described above. Candidate nucleotide sequences exhibiting significant increases or decreases in expression are identified and subjected to further analysis. An "identified nucleotide sequence" is intended to mean a candidate sequence that displays significant increases or decreases in its level of expression. Once such a nucleotide sequence is identified, the particular transcriptional regulatory region that is modulated by the identified nucleotide sequence is determined. In one embodiment, quantitative RT-PCR of the transiently expressed RNA pool extracted from the population of host cells is employed to identify the specific transcriptional regulatory region modulated by the identified nucleotide sequence. Primers specific to the identified nucleotide sequence and to a 5' UTR sequence of one of the populations of polynucleotide constructs may be used in quantitative RT-PCR methods.

Compositions that can be used to practice the methods of the claimed invention are further provided. In one embodiment, the invention provides isolated libraries, each of which comprises a population of polynucleotide constructs. Each polynucleotide construct in an isolated library comprises a common transcriptional regulatory region operably linked to a candidate nucleotide sequence. In some embodiments, the transcriptional regulatory region comprises an inducible promoter, such as, for example, the maize bronze-1 promoter. The polynucleotide constructs of the library may further comprise an intron, such as, for example, maize Ubil-int1. In one aspect of the invention, each polynucleotide construct in the library further comprises a common 5' UTR sequence.

The isolated libraries and populations of polynucleotide constructs of the present invention may also be used as reagents in kits. For example, kits of the invention can be employed in the various methods disclosed herein. In one embodiment, the kit comprises a population of polynucleotide constructs, wherein each of said polynucleotide constructs comprises a common transcriptional regulatory region operably linked to a candidate nucleotide sequence. The kit can further include a complementary DNA microarray.

In another embodiment, a kit of the invention comprises at least a first and a second population of polynucleotide constructs. The polynucleotide constructs of each population comprise a common transcriptional regulatory region operably linked to a candidate nucleotide sequence and further comprise a common 5' UTR sequence. The transcriptional regulatory regions and the 5' UTR sequences are unique to each population of polynucleotide constructs. The kit may further comprise a complementary DNA microarray. Any kit of the invention can further be accompanied by instructions for use.

The polynucleotide constructs of the invention will include in the 5'-3' direction of transcription, a transcriptional and translational initiation region (i.e., a transcriptional regulatory region), a candidate nucleotide sequence, and a transcriptional and translational termination region (i.e., termination region) functional in the host cell. The transcriptional regulatory region may be native or analogous, or foreign or heterologous, to the host cell and/or to the candidate nucleotide sequence. Additionally, the transcriptional regulatory region may be the natural sequence or alternatively a synthetic sequence. Where the transcriptional region is "foreign" or "heterologous" to the host cell, it is intended that the transcriptional regulatory region is not found in the native host cell into which it is introduced. Where the transcriptional regulatory region is "foreign" or "heterologous" to the candidate nucleotide sequence, it is intended that the transcriptional regulatory region is not the native or naturally occurring transcriptional regulatory region for the operably linked candidate nucleotide sequence.

The termination region may be native with the transcriptional regulatory region, may be native with the operably linked candidate nucleotide sequence, may be native with the host cell, or may be derived from another source (i.e., foreign or heterologous to the transcriptional regulatory region, the candidate sequence, the host cell, or any combination thereof). Convenient termination regions are available from the Ti-plasmid of *A. tumefaciens,* such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev 5: 141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acid Res. 15:9627-9639. In one embodiment, the PinII terminator is used.

Where appropriate, the polynucleotide constructs of the invention may be optimized for increased expression in the transformed host. That is, a sequence can be synthesized using host-preferred codons for improved expression. See, for example, Campbell and Gowri (1990) Plant Physiol. 92:1-11 for a discussion of host-preferred codon usage. In one embodiment, plant-preferred codons are utilized for improved expression in a plant host. Methods are available in the art for synthesizing plant-preferred genes. See, for example, U.S. Patent Nos. 5,380,831, and 5,436,391, and Murray et al. (1989) Nucleic Acids Res. 17:477-498.

Additional sequence modifications are known to enhance gene expression in a cellular host. These include elimination of sequences encoding spurious polyadenylation signals, exon-intron splice site signals, transposon-like repeats, and other such well-characterized sequences that may be deleterious to gene expression. The G-C content of the sequence may be adjusted to levels average for a given cellular host, as calculated by reference to known genes expressed in the host-cell. When possible, the sequence is modified to avoid predicted hairpin secondary mRNA structures.

The polynucleotide constructs of the invention may additionally contain 5' leader sequences. Such leader sequences can act to enhance translation. Translation leaders are known in the art and include: picornavirus leaders, for example, EMCV leader (Encephalomyocarditis 5' noncoding region) (Elroy-Stein et al. (1989) Proc. Natl. Acad Sci. USA 86:6126-6130); potyvirus leaders, for example, TEV leader (Tobacco Etch Virus) (Gallie et al. (1995) Gene 165(2):233-238), MDMV leader (Maize Dwarf Mosaic Virus), and human immunoglobulin heavy-chain binding protein (BiP) (Macejak et al. (1991) Nature 353:90-94); untranslated leader from the coat protein mRNA of alfalfa mosaic virus (AMV RNA 4) (Jobling et al. (1987) Nature 325:622-625); tobacco mosaic virus leader (TMV) (Gallie et al. (1989) in Molecular Biology of RNA, ed. Cech (Liss, New York), pp. 237-256); and maize chlorotic mottle virus leader (MCMV) (Lommel et al. (1991) Virology 81:382-385). See also, Della-Cioppa et al. (1987) Plant Physiol. 84:965-968..

The polynucleotide constructs of the invention may further comprise mRNA stabilizing sequences. An "mRNA stabilizing sequence" is intended to mean sequences, such as polyadenylation signals, that increase the half-life of mRNA molecules in the cell. In the methods of the present invention, the DNA encoding such mRNA stabilizing sequences is operably linked to the candidate nucleotide sequence. Suitable polyadenylation signals include those polyadenylation signals that can be modified without loss of transcript processing or stability, such as a 315 nucleotide fragment of the potato proteinase inhibitor II terminator region (GenBank Accession Number X04118) or the 315 nucleotide PinII terminator. One of skill in the art will recognize other stabilizing sequences that can be used in the methods of the invention.

In preparing the polynucleotide constructs of the invention, the various DNA fragments may be manipulated, so as to provide for the DNA sequences in the proper orientation and, as appropriate, in the proper reading frame. Toward this end, adapters or linkers may be employed to join the DNA fragments or other manipulations may be involved to provide for convenient restriction sites, removal of superfluous DNA, removal of restriction sites, or the like. For this purpose, *in vitro* mutagenesis, primer repair, restriction, annealing, resubstitutions, e.g., transitions and transversions, may be involved.

Any transcriptional regulatory region can be used in the practice of the invention, including a number of promoters well known in the art. The promoters can be selected based on the desired outcome. Complete or minimal promoter sequences can be used in the present invention. Promoters may be derived from any source, including, for example, prokaryotic or eukaryotic cells, or may be recombinantly engineered sequences. Constitutive promoters include, for example, the core promoter of the Rsyn7 promoter and other constitutive promoters disclosed in WO 99/43838 and U.S. Patent No. 6,072,050; the core CaMV 35S promoter (Odell et al. (1985) Nature 313:810-812); rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); ALS promoter (U.S. Patent No. 5,659,026), and the like. Other constitutive promoters include, for example, U.S. Patent Nos. 5,608,149; 5,608,144; 5,604,121; 5,569,597; 5,466,785; 5,399,680; 5,268,463; and 5,608,142.

While any transcriptional regulatory region may be used in the invention, one of skill in the art would recognize that the particular transcriptional regulatory region chosen will affect the screening method needed to detect alterations in-expression level. For example, if transcriptional activators of a strong constitutive promoter are to be identified, the sensitivity of the selected detection method must permit the detection of increases in transcriptional activity above the high background activity of the promoter. Similarly, if transcriptional modulators that decrease the activity of a weak promoter are to be identified, the selected detection technique will be sensitive enough to detect decreases in transcriptional activity below the low background activity of the promoter.

Pathogen-inducible promoters would be beneficial for study of the pathogen response pathway in plants. Such promoters include those from pathogenesis-related proteins (PR proteins), which are induced following infection by a pathogen; e.g., PR proteins, SAR proteins, beta-1,3-glucanase, chitinase, etc. See, for example, Redolfi et al. (1983) Neth. J. Plant Pathol. 89:245-254; Uknes et al. (1992) Plant Cell 4:645-656; and Van Loon (1985) Plant Mol. Virol. 4:111-116*.* See also WO 99/43819, published Sept. 9, 1999.

Of interest are promoters that are expressed locally at or near the site of pathogen infection. See, for example, Marineau et al. (1987) Plant Mol. Biol. 9:335-342; Matton et al. (1989) Molecular Plant-Microbe Interactions 2:325-331; Somsisch et al. (1986) Proc. Natl. Acad. Sci. USA 83:2427-2430; Somsisch et al. (1988) Mol. Gen. Genet. 2:93-98; and Yang (1996) Proc. Natl. Acad. Sci. USA 93:14972-14977. See also, Chen et al. (1996) Plant J. 10:955-966; Zhang et al. (1994) Proc. Natl. Acad. Sci. USA 91:2507-2511; Warner et al. (1993) Plant J. 3:191-201; Siebertz et al. (1989) Plant Cell 1:961-968; U.S. Patent No. 5,750,386 (nematode-inducible); and the references cited therein. Of particular interest is the inducible promoter for the maize PRms gene, whose expression is induced by the pathogen *Fusarium moniliforme* (see, for example, Cordero et al. (1992) Physiol. Mol. Plant Path. 41:189-200). In an embodiment, a defense inducible promoter operably linked to a U-TAG in the first DNA construct is used for high through put analysis of expression of candidate R genes.

Wound-inducible promoters would be beneficial for the study of the plant response to physical damage or wounds. Such wound-inducible promoters include potato proteinase inhibitor (pin II) gene (Ryan (1990) Ann. Rev. Phytopath. 28:425-449; Duan et al. (1996) Nature Biotechnology 14:494-498); wun1 and wun2, U.S. Patent No. 5,428,148; win1 and win2 (Stanford et al. (1989) Mol. Gen. Genet. 215:200-208); systemin (LcGur1 et al. (1992) Science 225:1570-1573); WIP1 (Rohmeier et al. (1993) Plant Mol. Biol. 22:783-792; Eckelkamp et al. (1993) FEBS Letters 323:73-76); MPI gene (Corderok et al. (1994) Plant J. 6(2):141-150).

Chemically regulated promoters would be beneficial for the study of the responses to elicitor compounds. Chemical-regulated promoters can be used to modulate the expression of a gene in a plant through the application of an exogenous chemical regulator. Depending upon the objective, the promoter may be a chemical-inducible promoter, where application of the chemical induces gene expression, or a chemical-repressible promoter, where application of the chemical represses gene expression. Chemical-inducible promoters are known in the art and include, but are not limited to, the maize In2-2 promoter, which is activated by benzenesulfonamide herbicide safeners, the maize GST promoter, which is activated by hydrophobic electrophilic compounds that are used as pre-emergent herbicides, and the tobacco PR-1a promoter, which is activated by salicylic acid. Other chemical-regulated promoters of interest include steroid-responsive promoters (see, for example, the glucocorticoid-inducible promoter in Schena et al. (1991) Proc. Natl. Acad. Sci. USA 88:10421-10425 and McNellis et al. (1998) Plant J. 14(2):247-257) and tetracycline-inducible and tetracycline-repressible promoters (see, for example, Gatz et al. (1991) Mol. Gen. Genet. 227:229-237, and U.S. Patent Nos. 5,814,618 and 5,789,156).

Other inducible promoters of interest include the maize bronze-1 promoter (see, for example, Roth et al..(1991) Plant Cell 3: 317-323; Furtek et al. (1988) Plant Mol. Biol. 11: 473-481; Ralston et al. (1988) Genetics 119: 185-197); E-selectin promoter, the migA promoter (Yang et al. (2000) Microbiology 146:2509-2519), Ipc1, NF-κB, heavy metal-inducible human metallothionein IIA promoter, P1 and P3 of *Pseudomonas aeruginosa* (Schurr et al. (1995) J. Bacteriol 177:5670-5679*).* Commonly used promoters for expression in mammalian cells are derived from polyoma, Adenovirus 2, cytomegalovirus, and Simian Virus 40. For other suitable expression systems for both prokaryotic and eukaryotic cells, see chapters 16 and 17 of Sambrook et al. (1989) Molecular Cloning: A Laboratory Manual (2d ed., Cold Spring Harbor Laboratory Press, Plainview, New York). See, Goeddel (1990) in Gene Expression Technology: Methods in Enzymology 185 (Academic Press, San Diego, California).

Tissue-preferred promoters would be beneficial for the study of differentiation or development. Tissue-preferred promoters include Yamamoto et al. (1997) Plant J. 12(2):255-265; Kawamata et al. (1997) Plant Cell Physiol. 38(7):792-803; Hansen et al. (1997) Mol. Gen Genet. 254(3):337-343; Russell et al. (1997) Transgenic Res. 6(2):157-168; Rinehart et al. (1996) Plant Physiol. 112(3):1331-1341; Van Camp et al. (1996) Plant Physiol. 112(2):525-535; Canevascini et al. (1996) Plant Physiol. 112(2):513-524; Yamamoto et al. (1994) Plant Cell Physiol. 35(5):773-778; Lam (1994) Results Probl. Cell Differ. 20:181-196; Orozco et al. (1993) Plant Mol Biol. 23(6):1129-1138; Matsuoka et al. (1993) Proc Natl. Acad. Sci. USA 90(20):9586-9590; and Guevara-Garcia et al. (1993) Plant J. 4(3):495-505.

Suitable tissue-specific promoters include the albumin promoter (e.g., liver-specific promoter; Pinkert et al. (1987) Genes Dev. 1:268-277), lymphoid-specific promoters (Calame and Eaton (1988) Adv. Immunol. 43:235-275), in particular promoters of T-cell receptors (Winoto and Baltimore (1989) EMBO J. 8:729-733) and immunoglobulins (Banerji et al. (1983) Cell 33:729-740; Queen and Baltimore (1983) Cell 33:741-748), neuron-specific promoters (e.g., the neurofilament promoter; Byrne and Ruddle (1989) Proc. Natl. Acad. Sci. USA 86:5473-5477), pancreas-specific promoters (Edlund et al. (1985) Science 230:912-916), and mammary gland-specific promoters (e.g., milk whey promoter; U.S. Patent No. 4,873,316 and European Application Patent Publication No. 264,166). Developmentally regulated promoters are also encompassed, for example the murine hox homeobox promoters (Kessel and Gruss (1990) Science 249:374-379), the α-fetoprotein promoter (Campes and Tilghman (1989) Genes Dev. 3:537-546), and the like. Such promoters can be modified, if necessary, for weak expression.

Leaf-preferred promoters are known in the art. See, for example, Yamamoto et al. (1997) Plant J. 12(2):255-265; Kwon et al. (1994) Plant Physiol. 105:357-67*;* Yamamoto et al. (1994) Plant Cell Physiol. 35(5):773-778; Gotor et al. (1993) Plant J. 3:509-18; Orozco et al. (1993) Plant Mol. Biol. 23(6):1129-1138; and Matsuoka et al. (1993) Proc. Natl. Acad. Sci. USA 90(20):9586-9590. In addition, promoter of cab and rubisco can also be used. See, for example, Stockinger and Walling (1994) Plant Physiol. 104:1475-1476 and Timko et al. (1985) Nature 318:579-582.

Root-preferred promoters are known and can be selected from the many available from the literature or isolated de novo from various compatible species. See, for example, Hire et al. (1992) Plant Mol. Biol. 20(2):207-218 (soybean root-specific glutamine synthetase gene); Keller and Baumgartner (1991) Plant Cell 3(10):1051-1061 (root-specific control element in the GRP 1.8 gene of French bean); Sanger et al. (1990) Plant Mol. Biol. 14(3):433-443 (root-specific promoter of the mannopine synthase (MAS) gene of *Agrobacterium tumefaciens*)*;* and Miao et al. (1991) Plant Cell 3(1):11-22 (full-length cDNA clone encoding cytosolic glutamine synthetase (GS), which is expressed in roots and root nodules of soybean). See also Bogusz et al. (1990) Plant Cell 2(7):633-641, where two root-specific promoters isolated from hemoglobin genes from the nitrogen-fixing nonlegume *Parasponia andersonii* and the related non-nitrogen-fixing nonlegume *Trema tomentosa* are described. The promoters of these genes were linked to a β-glucuronidase reporter gene and introduced into both the nonlegume *Nicotiana tabacum* and the legume *Lotus corniculatus,* and in both instances root-specific promoter activity was preserved. Leach and Aoyagi (1991) describe their analysis of the promoters of the highly expressed rolC and rolD root-inducing genes of *Agrobacterium rhizogenes* (see Plant Science (Limerick) 79(1):69-76). They concluded that enhancer and tissue-preferred DNA determinants are dissociated in those promoters. Teeri *et al.* (1989) used gene fusion to lacZ to show that the *Agrobacterium* T-DNA gene encoding octopine synthase is especially active in the epidermis of the root tip and that the TR2' gene is root specific in the intact plant and stimulated by wounding in leaf tissue, an especially desirable combination of characteristics for use with an insecticidal or larvicidal gene (see EMBO J. 8(2):343-350). The TR1' gen fused to *nptII* (neomycin phosphotransferase II) showed similar characteristics. Additional root-preferred promoters include the VfENOD-GRP3 gene promoter (Kuster et al. (1995) Plant Mol. Biol. 29(4):759-772); and rolB promoter (Capana et al. (1994) Plant Mol. Biol. 25(4):681-691. See also U.S. Patent Nos. 5,837,876; 5,750,386; 5,633,363; 5,459,252; 5,401,836; 5,110,732; and 6,023,179. The phaseolin gene (Murai et al. (1983) Science 23:476-482 and Sengopta-Gopalen et al. (1988) PNAS 82:3320-3324.

"Seed-preferred" promoters include both "seed-specific" promoters (those promoters active during seed development such as promoters of seed storage proteins) as well as "seed-germinating" promoters (those promoters active during seed germination). See Thompson et al. (1989) BioEssays 10:108. Such seed-preferred promoters include, but are not limited to, Cim1 (cytokinin-induced message); cZ19B1 (maize 19 kDa zein); and milps (myo-inositol-1-phosphate synthase); (see WO 00/11177 and U.S. Patent No. 6,225,529). Gamma-zein is an endosperm-specific promoter. Glb-1 is an embryo-specific promoter. See, for example, Yang et al. (2001) Proc. Natl. Acad. Sci. U. S. A. 98(20):11438-43. For dicots, seed-specific promoters include, but are not limited to, bean β-phaseolin, napin, β-conglycinin, soybean lectin, cruciferin, and the like. For monocots, seed-specific promoters include, but are not limited to, maize 15 kDa zein, 22 kDa zein, 27 kDa zein, g-zein, waxy, shrunken 1, shrunken 2, globulin 1, etc. See also WO 00/12733, where seed-preferred promoters from *end1* and *end2* genes are disclosed.

Depending on the desired results, weak promoters may be beneficial. Where low level expression is desired, weak promoters will be used. Generally, a "weak promoter" is intended to mean a promoter that drives expression of a coding sequence at a low level. A "low level" is intended to mean at levels of about 1/1000 transcripts to about 1/100,000 transcripts to about 1/500,000 transcripts. Alternatively, it is recognized that weak promoters also encompass promoters that are expressed in only a few cells and not in others to give a total low level of expression. Where a promoter is expressed at unacceptably high levels, portions of the promoter sequence can be deleted or modified to decrease expression levels.

Such weak constitutive promoters include, for example, the core promoter of the Rsyn7 promoter (WO 99/43838 and U.S. Patent No. 6,072,050), the core 35S CaMV promoter, and the like. Other constitutive promoters include, for example, U.S. Patent Nos. 5,608,149; 5,608,144; 5,604,121; 5,569;597; 5,466,785; 5,399,680; 5,268,463; and 5,608,142. See also, U.S. Patent NO. 6,177,611.

In accordance with the methods of the invention, the polynucleotide constructs described herein are introduced into any host cell of interest. "Introducing" a population of polynucleotide constructs is intended to mean presenting to the host cells a population of polynucleotide constructs in such a manner that the constructs gain access to the interior of a host cell. The methods of the invention do not depend on a particular method for introducing a polynucleotide construct into a host cell. Host cells of interest include but are not limited to both prokaryotic and eukaryotic cells, for example, mammalian and plant cells. In one embodiment, the host cells are plant cells. Methods of introducing nucleotide sequences into cells and tissues from various organisms are well known in the art and include, but are not limited to, stable transformation methods, transient transformation methods, and virus-mediated methods.

The polynucleotide constructs of the invention can be introduced into prokaryotic or eukaryotic cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride co-precipitation, DEAE-dextran-mediated transfection, lipofection, or electroporation. Suitable methods for transforming or transfecting host cells can be found in Sambrook et al. (1989) Molecular Cloning: A Laboratory Manual (2d ed., Cold Spring Harbor Laboratory Press, Plainview, New York) and other laboratory manuals. In some embodiments, plants or plant cells are transiently transformed with the polynucleotide constructs of the invention. "Transiently transformed" is intended to mean that a polynucleotide construct introduced into a plant does not integrate into the genome of the plant. In other embodiments, plants or plant cells are stably transformed with the polynucleotide constructs of the invention. "Stable transformation" is intended to mean that the nucleotide construct introduced into a plant integrates into the genome of the plant and is capable of being inherited by the progeny thereof.

The polynucleotide constructs of the invention can be transiently transformed into the plant using techniques know in the art. Such techniques include viral vector system and the precipitation of the polynucleotide in a manner that precludes subsequent release of the DNA. Thus, the transcription from the particle-bound DNA can occur, but the frequency with which its released to become integrated into the genome is greatly reduced. Such methods include the use particles coated with polyethylimine (PEI; Sigma #P3143).

In other embodiments, the polynucleotide of the invention may be introduced into plants by contacting plants with a virus or viral nucleic acids. Generally, such methods involve incorporating a nucleotide construct of the invention within a viral DNA or RNA molecule. It is recognized that promoters of the invention also encompass promoters utilized for transcription by viral RNA polymerases. Methods for introducing polynucleotides into plants and expressing a protein encoded therein, involving viral DNA or RNA molecules, are known in the art. See, for example, U.S. Patent Nos. 5,889,191, 5,889,190, 5,866,785, 5,589,367, -5,316,93 1, and Porta et aL (1996) Molecular Biotechnology 5:209-221.

Methods are known in the art for the targeted insertion of a polynucleotide at a specific location in the plant genome. In one embodiment, the insertion of the polynucleotide at a desired genomic location is achieved using a site-specific recombination system. See, for example, WO99/25821, WO99/25854, WO99/25840, WO99/25855, and WO99/25853. Briefly, the polynucleotide of the invention can be contained in transfer cassette flanked by two non-recombinogenic recombination sites. The transfer cassette is introduced into a plant having stably incorporated into its genome a target site that is flanked by two non-recombinogenic recombination sites that correspond to the sites of the transfer cassette. An appropriate recombinase is provided and the transfer cassette is integrated at the target site. The polynucleotide of interest is thereby integrated at a specific chromosomal position in the plant genome.

Transformation protocols as well as protocols for introducing nucleotide sequences into plants may vary depending on the type of plant or plant cell, i.e., monocot or dicot, targeted for transformation. Suitable methods of introducing nucleotide sequences into plant cells and subsequent insertion into the plant genome include microinjection (Crossway et al. (1986) Biotechniques 4:320-334), electroporation (Riggs et al. (1986) Proc. Natl. Acad. Sci. USA 83:5602-5606, *Agrobacterium*-mediated transformation (Townsend et al., U.S. Patent No. 5,563,055; Zhao et al., U.S. Patent No. 5,981,840), direct gene transfer (Paszkowski et al. (1984) EMBO J. 3:2717-2722), and ballistic particle acceleration (see, for example, Sanford et al., U.S. Patent No. 4,945,050; Tomes et al., U.S. Patent No. 5,879,918; Tomes et al., U.S. Patent No. 5,886,244; Bidney et al., U.S. Patent No. 5,932,782; Tomes et al. (1995) "Direct DNA Transfer into Intact Plant Cells via Microprojectile Bombardment," in Plant Cell, Tissue, and Organ Culture: Fundamental Methods, ed. Gamborg and Phillips (Springer-Verlag, Berlin); McCabe et al. (1988) Biotechnology 6:923-926); and Lec1 transformation (WO 00/28058). Also see Weissinger et al. (1988) Ann. Rev. Genet. 22:421-477; Sanford et al. (1987) Particulate Science and Technology 5:27-37 (onion); Christou et al. (1988) Plant Physiol. 87:671-674 (soybean); McCabe et al. (1988) Bio/Technology 6:923-926 (soybean); Finer and McMullen (1991) In Vitro Cell Dev. Biol. 27P:175-182 (soybean); Singh et al. (1998) Theor. Appl. Genet. 96:319-324 (soybean); Datta et al. (1990) Biotechnology 8:736-740 (rice); Klein et al. (1988) Proc. Natl. Acad. Sci. USA 85:4305-4309 (maize); Klein et al. (1988) Biotechnology 6:559-563 (maize); Tomes, U.S. Patent No. 5,240,855; Buising et al., U.S. Patent Nos. 5,322,783 and 5,324,646; Tomes et al. (1995) "Direct DNA Transfer into Intact Plant Cells via Microprojectile Bombardment," in Plant Cell, Tissue, and Organ Culture: Fundamental Methods, ed. Gamborg (Springer-Verlag, Berlin) (maize); Klein et al. (1988) Plant Physiol. 91:440-444 (maize); Fromm et al. (1990) Biotechnology 8:833-839 (maize); Hooykaas-Van Slogteren et al. (1984) Nature (London) 311:763-764; Bowen et al., U.S. Patent No. 5,736,369 (cereals); Bytebier et al. (1987) Proc. Natl. Acad. Sci. USA 84:5345-5349 (Liliaceae); De Wet et al. (1985) in The Experimental Manipulation of Ovule Tissues, ed. Chapman et al. (Longman, New York), pp. 197-209 (pollen); Kaeppler et al. (1990) Plant Cell Reports 9:415-418 and Kaeppler et al. (1992) Theor. Appl. Genet. 84:560-566 (whisker-mediated transformation); D'Halluin et al.. (1992) Plant Cell 4:1495-1505 (electroporation); Li et al. (1993) Plant Cell Reports 12:250-255 and Christou and Ford (1995) Annals of Botany 76:407-413 (rice); Osjoda et al. (1996) Nature Biotechnology 14:745-750 (maize via *Agrobacterium tumefaciens*).

The methods of the invention can be used to identify nucleotide sequences that modulate transcriptional regulatory regions of a physiological pathway of interest. A "physiological pathway of interest" is intended to mean any biological process that has a genetic component including but not limited to increased expression of a polypeptide, decreased expression of a polypeptide, increased transcription of a nucleotide sequence, decreased transcription of a nucleotide sequence, increased replication of a nucleotide sequence, and decreased replication of a nucleotide sequence. The physiological pathways of interest include but are not limited to, signal transduction; housekeeping; insect resistance; pathogen resistance; herbicide resistance; reproduction; sterility; carbohydrate, polypeptide, nutrient, oil, and starch metabolism; carbohydrate modifications; growth; detoxification; male or female gametophytic development; disease progression, cancer development, tissue and organ differentiation; tissue, organ, and organism development; apoptosis; toxicity; cell senescence; recombination; mutagenesis; DNA repair; stress response; heat shock response; osmotic response; angiogenesis; congenital disorders; replication; transcription; translation; R-gene mediated response; and pathogen response.

A "pathogen resistance pathway" is intended to mean any pathway that responds to pathogen invasion or attack including, but not limited to, R-gene mediated response and the HR response. A "tissue developmental pathway" is intended to mean any pathway that is involved in the development or differentiation of a tissue, including but not limited to, bud formation, root formation, apical growth, and organogenesis in a plant or animal. A "metabolic pathway" is intended to mean any pathway that is involved in cellular metabolism, including but not limited to, nutrient utilization, amino acid biosynthesis, respiration, photosynthesis, replication, membrane expansion, cell wall expansion, osmotic regulation, and catabolism. An "apoptotic" pathway is intended to mean any pathway involved in the process of apoptosis or programmed cell death, including but not limited to, genome degradation, p53, TNF-α, poly ADP-ribose polymerase degradation, and Ca²⁺ influx.

The method can be used to investigate physiological pathways of interest in numerous cell types, including but not limited to plant, animal, mammalian, fungal, and bacterial cells. Suitable mammalian cells include, but are not limited to, Chinese hamster ovary cells (CHO) or COS cells. Suitable tissue types include, but are not limited to, healthy and diseased lung, spleen, brain, colon, liver, skin, thyroid, uterus, endometrium, ovary, prostate, breast, immune cells, bone marrow, heart, nerve, blood vessel, thymus, kidney, testis, muscle, pancreas, and small intestine.

As used herein, the term "plant cell" includes, without limitation, seeds, suspension cultures, embryos, meristematic regions, callus tissue, leaves, roots, shoots, gametophytes, sporophytes, pollen, and microspores. One particular tissue for high throughput assays in maize is Black Mexican Sweet (BMS) tissue cultures. However, maize tissues from other sources can be used. A number of plant tissues can be used, including, but not limited to, immature embryos, infiltrated leaves, hypocotyls, or root tissues.

The present invention may be used to investigate transcriptional regulation in any plant species, including, but not limited to, monocots and dicots. Examples of plant species of interest include, but are not limited to, corn (Zea *mays*)*, Brassica* sp. (e.g., *B. napus, B. rapa, B. juncea*)*,* particularly those *Brassica* species useful as sources of seed oil, alfalfa (*Medicago sativa*)*,* rice (*Oryza sativa*)*,* rye (*Secale cereale*)*,* sorghum (*Sorghum bicolor, Sorghum vulgare*)*,* millet (e.g.; pearl millet (*Pennisetum glaucum*)*,* proso millet (*Panicum miliaceum*)*,* foxtail millet (*Setaria italica*)*,* finger millet (*Eleusine coracana*))*,* sunflower *(Helianthus annuus),* safflower (*Carthamus tinctorius*)*,* wheat (*Triticum aestivum*)*,* soybean (*Glycine max*)*,* tobacco (*Nicotiana tabacum*)*,* potato (*Solanum tuberosum*)*,* peanuts (*Arachis hypogaea*)*,* cotton (*Gossypium barbadense, Gossypium hirsutum*)*,* sweet potato (*Ipomoea batatus*)*,* cassava (*Manihot esculenta*)*,* coffee (*Coffea* spp*.*), coconut (*Cocos nucifera*)*,* pineapple (*Ananas comosus*)*,* citrus trees (*Citrus* spp.), cocoa (*Theobroma cacao*)*,* tea (*Camellia sinensis*)*,* banana (Musa spp.), avocado (*Persea americana*)*,* fig (*Ficus casica*)*,* guava (*Psidium guajava*)*,* mango (*Mangifera indica*)*,* olive (*Olea europaea*)*,* papaya (*Carica papaya*)*,* cashew (*Anacardium occidentale*)*,* macadamia (*Macadamia integrifolia*)*,* almond (Prunus *antygdalus*)*,* sugar beets (*Beta vulgaris*)*,* sugarcane (*Saccharum* spp.), oats, barley, vegetables, ornamentals, and conifers.

Vegetables include tomatoes (*Lycopersicon esculentum*)*,* lettuce·(e.g., *Lactuca sativa*)*,* green beans (*Phaseolus vulgaris*)*,* lima beans (*Phaseolus limensis*)*,* peas (*Lathyrus* spp.), and members of the genus *Cucumis* such as cucumber·(C. *sativus*)*,* cantaloupe (C. *cantalupensis),* and musk melon (C. *melo*)*.* Ornamentals include azalea (*Rhododendron* spp.), hydrangea (*Macrophylla hydrangea)*, hibiscus(Hibiscus *rosasanensis*)*,* roses (*Rosa* spp.), tulips (*Tulipa* spp.), daffodils (*Narcissus* spp.), petunias (*Petunia hybrida*)*,* carnation (*Dianthus caryophyllus*)*,* poinsettia (*Euphorbia pulcherrima*)*,* and chrysanthemum.

Conifers that may be employed in practicing the present invention include, for example, pines such as loblolly pine (*Pinus taeda*)*,* slash pine (*Pinus elliotii*)*,* ponderosa pine (*Pinus ponderosa*)*,* lodgepole pine (*Pinus contorta*)*,* and Monterey pine (*Pinus radiata*)*;* Douglas-fir (*Pseudotsuga menziesii*)*;* Western hemlock (*Tsuga canadensis*)*;* Sitka spruce (*Picea glauca*)*;* redwood (*Sequoia sempervirens*)*;* true firs such as silver fir (*Abies amabilis*) and balsam fir (*Abies balsamea*)*;* and cedars such as Western red cedar (*Thuja plicata*) and Alaska yellow-cedar (*Chamaecyparis nootkatensis*)*.* Optimally, plants of the present invention are crop plants (for example, corn, alfalfa, sunflower, *Brassica,* soybean, cotton, safflower, peanut, sorghum, wheat, millet, tobacco, etc.), more optimally corn and soybean plants, yet more optimally corn plants.

Plants of particular interest include grain plants that provide seeds of interest, oil-seed plants, and leguminous plants. Seeds of interest include grain seeds, such as corn, wheat, barley, rice, sorghum, rye, etc. Oil-seed plants include cotton, soybean, safflower, sunflower, *Brassica,* maize, alfalfa, palm, coconut, etc. Leguminous plants include beans and peas. Beans include guar, locust bean, fenugreek, soybean, garden beans, cowpea, mungbean, lima bean, fava bean, lentils, chickpea, etc.

As used herein, "nucleic acid" or "nucleotide sequence" or "polynucleotide" includes reference to a deoxyribonucleotide or ribonucleotide polymer in either single- or double-stranded form, and unless otherwise limited, encompasses known analogues (*e.g*., peptide nucleic acids) having the essential nature of natural nucleotides in that they hybridize to single-stranded nucleic acids in a manner similar to naturally occurring nucleotides.

The use of the terms "polynucleotide constructs" or "nucleotide constructs" herein is not intended to limit the present invention to nucleotide constructs comprising DNA. Those of ordinary skill in the art will recognize that nucleotide constructs, particularly polynucleotides and oligonucleotides composed of ribonucleotides and combinations of ribonucleotides and deoxyribonucleotides, may also be employed in the methods and compositions disclosed herein. The nucleotide constructs, nucleic acids, and nucleotide sequences of the invention additionally encompass all complementary forms of such constructs, molecules, and sequences. Further, the nucleotide constructs, nucleotide molecules, and nucleotide sequences of the present invention encompass all nucleotide constructs, molecules, and sequences which can be employed in the methods of the present invention for transforming a host cell of interest including, but not limited to, those comprised of deoxyribonucleotides, ribonucleotides, and combinations thereof. Such deoxyribonucleotides and ribonucleotides include both naturally occurring molecules and synthetic analogues. The nucleotide constructs, nucleic acids, and nucleotide sequences of the invention also encompass all forms of nucleotide constructs including, but not limited to, single-stranded forms, double-stranded forms, hairpins, stem-and-loop structures, and the like.

The terms "polypeptide," "peptide," and "protein" are used interchangeably herein to refer to a polymer of amino acid residues. The terms apply to amino acid polymers in which one or more amino acid residues is an artificial chemical analogue of a corresponding naturally occurring amino acid, as well as to naturally occurring amino acid polymers.

As used herein, the terms "encoding" or "encoded" when used in the context of a specified nucleic acid mean that the nucleic acid comprises the requisite information to direct translation of the nucleotide sequence into a specified protein. The information by which a protein is encoded is specified by the use of codons. A nucleic acid encoding a protein may comprise non-translated sequences (*e.g*., introns) within translated regions of the nucleic acid or may lack such intervening non-translated sequences (*e.g*., as in cDNA).

It should also be noted that, as used in this specification and the appended embodiments, the singular forms "a," "an," and "the" include the plural referents unless the context clearly dictates otherwise. Thus, for example, reference to a method for identifying "a nucleotide sequence" includes a method for identifying more than one nucleotide sequence that encodes a polypeptide that modulates the activity of a transcriptional regulatory region.

Units, prefixes, and symbols may be denoted in their SI accepted form. Unless otherwise indicated, nucleic acids are written left to right in 5' to 3' orientation; amino acid sequences are written left to right in amino to-carboxy orientation, respectively. Numeric ranges are inclusive of the numbers defining the range. Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes. The above-defined terms are more fully defined by reference to the specification as a whole.

Throughout the specification the word "comprising," or variations such as "comprises" or "comprising," will be understood to imply the inclusion of a stated element, integer or step, or group of elements, integers or steps, but not the exclusion of any other element, integer or step, or group of elements, integers or steps.

The following examples are presented by way of illustration, not by way of limitation.

### EXPERIMENTAL

### Example 1: Agrobacterium-mediated Transformation of Maize Cells

For *Agrobacterium-mediated* transformation of maize cells with a population of polynucleotide constructs of the invention, optimally the following method is used. Media recipes follow. *Agrobacterium tumefaciens* cells were cultured on solid 800 medium and incubated at 27°C in the dark for one day. A single colony was transferred to solid 810 medium and incubated at 27°C in the dark for two days. *Agrobacterium* from the 810 plate was suspended in #561Q liquid medium containing 0.1 mM acetosyringone to a density of O.D550nm=0.25, at which point it was ready for co-cultivation with BMS cells.

Black Mexican Sweet (BMS) maize cells were maintained in #237 medium. The cells were collected by gravity. Under a tissue culture hood, the supernatant was removed and the BMS cell pellet was washed three times with #561Q + 0.1 mM acetosyringone. The pellet from the last wash was diluted at a ratio of 1:3 (v/v) with #561Q + 0.1 mM acetosyringone and was then ready for co-cultivation with *Agrobacterium.*

### Co-cultivation of BMS cells with Agrobacterium

Seven mL of BMS cells were mixed with 1 mL of the resuspended *Agrobacterium* cells. The BMS cells were co-cultivated with the *Agrobacterium* cells on a gyratory shaker at 140 rpm for 3 hours at 27°C in the dark. The cells were collected by gravity and plated onto a glass-filter placed over 562P solid medium. The mixture was incubated at 27°C in the dark for two days, at which point a 48 hour time point sample is taken. Filters were then moved onto #563N medium, and another sample was taken at 72 hours.

### Media Recipes

562P comprises 4 g/L Chu(N6) Basal Salts (Sigma C-1416), 1 mL/L Eriksson's Vitamin Mix (1000X Sigma-1511), 0.5 mL/L Thiamine HCl, 2 mL/L 2,4-dichlorophenoxyacetic acid, 0.69 g/L L-Proline, 30 g/L sucrose, 3 g/L gelrite, 0.85 mL/L silver nitrate, and 1 mL/L of 100 mM acetosyringone at pH 5.8.

561Q comprises 4 g/L Chu(N6) Basal Salts (Sigma C-1416), 1 mL/L Eriksson's Vitamin Mix (1000X Sigma-1511), 0.5 mL/L Thiamine HCl, 1.5 mL/L 2,4- dichlorophenoxyacetic acid, 0.69 g/L L-Proline, 68.5 g/L sucrose, and 36 g/L glucose at pH 5.3.

563N comprises 4 g/L Chu(N6) Basal Salts (Sigma C-1416), 1 mL/L Eriksson's Vitamin Mix (1000X Sigma-1511), 0.5 mL/L Thiamine HCl, 1.5 mL/L 2,4- dichlorophenoxyacetic acid, 0.69 g/L L-Proline, 30 g/L sucrose, 0.5 g/L MES buffer, 8.0 g/L purified agar (Sigma), 0.85 mL/L Silver Nitrate, and 100 mL/L (AgriBio) Carbenicillin at pH 5.8.

#237 comprises 4.3 g/L MS Salts (Gibco 11117), 0.1 g/L myo-inositol, 5 mL/L MS Vitamin Stock Solution, 2 mL/L 2,4- dichlorophenoxyacetic acid, and 30 g/L sucrose at pH 5.6.

MS Vitamin Stock Solution (36J) comprises 0.1 g/L nicotinic acid, 0.02 g/L thiamine-HCl, 0.1 g/L pyridoxine-HCl, and 0.4 g/L glycine brought to volume with polished D-I H20.

### Example 2: Transformation of Maize with Known Transcription Factors

Experiments using polynucleotide constructs comprising known transcription factors were performed to demonstrate that the methods of the invention can be used to screen nucleotide sequences to identify sequences that modulate a transcriptional regulatory region. Specifically, two polynucleotide constructs were constructed that comprise the inducible maize bronze-1 (Bz1) promoter operably linked to either CRC or P, two previously identified transcriptional factors known to be an inducer and a non-inducer of the Bz1 promoter, respectively. Bruce et al. (2000) Plant Cell 12: 65-80. The Bz1 promoter was operably linked to either CRC or P via the maize intron Ubi1-int1. This intron increases the background expression of the nucleotide sequence operably linked to the Bz1 promoter while preserving the promoter's sensitivity to further stimulation by an activator of transcription. Both polynucleotide constructs were then transferred to JT *Agro* expression plasmids, and two independent transformations of BMS cells were performed, as described in Example 1.

Transient expression of CRC and P was monitored over several days using standard RT-PCR methods. A significant increase in expression of the Bz1 promoter activator CRC was observed between 48 and 72 hours. Cells expressing the non-inducer P, however, did not display a significant increase or decrease in the amount of P transcript over the same time period. Thus, expression of a nucleotide sequence that is known to increase the activity of the Bz1 promoter stimulated further expression of the sequence itself via a positive feedback loop. In contrast, no significant change in the expression level of a nucleotide sequence that does not activate the Bz1 promoter was observed. These results confirm that the methods of the present invention can be used to identify nucleotide sequences that modulate the activity of a transcriptional regulatory region.

### Example 3: Identifying Modulators of Gene Transcription

Candidate nucleotide sequences are PCR-amplified from a maize cDNA library using attB-flanked gene-specific primers, according to methods well known in the art. Invitrogen's Gateway^{™} recombination system is used to generate a population of polynucleotide constructs comprising the Bz1 promoter operably linked to a candidate nucleotide sequence via the maize intron Ubi1-int1. The polynucleotide constructs are then introduced into a population of maize BMS cells by Agrobacterium-mediated transformation, as described in Example 1. Gene expression of candidate nucleotide sequences is compared at 48 h and 72 h post transformation. Specifically, cells are harvested *en masse,* RNA is collected, and the poly(A) RNA fraction is extracted. Labeled complementary RNA (cRNA) probes are synthesized from the isolated poly(A) RNA according to standard Agilent Technologies™ protocols (see, for example, the Agilent Technologies™ manual for "Low RNA Input Fluorescent Linear Amplification Kit"), with the exception that reverse transcription is performed with T7-promoter transgene-specific primers to selectively amplify the poly(A) RNA derived from the population of polynucleotide constructs. Expression levels of each candidate nucleotide sequence at 48h and 72 h are determined by hybridization of these labeled cRNA probes to arrays of oligonucleotides (e.g., Agilent™ nucleic acid arrays). Levels of expression at the two time points are compared to identify significant increases or decreases in candidate nucleotide expression.

All publications and patent applications mentioned in the specification are indicative of the level of those skilled in the art to which this invention pertains.

Although the foregoing invention has been described in some detail by way of illustration and example for purposes of clarity of understanding, it will be obvious that certain changes and modifications may be practiced within the scope of the embodiments described herein.

## Claims

1. A method for identifying a nucleotide sequence that encodes a polypeptide that modulates the activity of a transcriptional regulatory region, said method comprising:
(a) providing a population of polynucleotide constructs, wherein each of said polynucleotide constructs in said population comprises the transcriptional regulatory region operably linked to a candidate nucleotide sequence;
(b) introducing said population of polynucleotide constructs into a population of host cells; and,
(c) screening for a modulation in the expression level of each candidate nucleotide sequence, and thereby identifying a nucleotide sequence that encodes a polypeptide that modulates the activity of said transcriptional regulatory region.

2. The method of claim 1, wherein said screening for a modulation in the expression level of each candidate nucleotide sequence comprises:
(a) isolating mRNA from said population of host cells,
(b) preparing labeled polynucleotides from said isolated mRNA; and,
(c) hybridizing said labeled polynucleotides to a complementary DNA microarray.

3. The method of claim 2, wherein said isolating mRNA from said population of host cells allows for preferential amplification of mRNA derived from said population of polynucleotide constructs.

4. The method of claim 1, wherein said modulation comprises an increase in the expression level of a candidate nucleotide sequence.

5. The method of claim 1, wherein said modulation comprises a decrease in the expression level of a candidate nucleotide sequence.

6. The method of claim 1, wherein said transcriptional regulatory region comprises an inducible promoter.

7. The method of claim 6, wherein each of said polynucleotide constructs further comprises an intron.

8. The method of claim 7, wherein said transcriptional regulatory region has minimal background activity.

9. The method of claim 8, wherein said intron increases the background expression of said candidate nucleotide sequence.

10. The method of claim 9, wherein said intron is maize Ubi1-intron1.

11. The method of claim 10, wherein said inducible promoter is maize bronze-1 promoter.

12. The method of claim 1, wherein at least one of said candidate nucleotide sequences encodes a polypeptide that directly modulates the activity of said transcriptional regulatory region.

13. The method of claim 1, wherein at least one of said candidate nucleotide sequences encodes a polypeptide that indirectly modulates the activity of said transcriptional regulatory region.

14. The method of claim 1, wherein said host cells are plant cells.

15. The method of claim 14, wherein said plant cells are dicotyledonous.

16. The method of claim 14, wherein said plant cells are monocotyledonous.

17. The method of claim 14, wherein said plant cells are selected from the group consisting of maize, wheat, sorghum, rice, barley, soybean, alfalfa, sunflower, *Brassica,* and tomato.

18. The method of claim 1, wherein each of said candidate nucleotide sequences is from a plant cell.

19. The method of claim 2, wherein said transcriptional regulatory region regulates expression of a nucleotide sequence involved in a physiological pathway of interest, wherein said physiological pathway of interest is selected from the group consisting of a pathogen resistance pathway, a tissue developmental pathway, a metabolic pathway, and an apoptotic pathway.

20. A method for identifying a nucleotide sequence that encodes a polypeptide that modulates the activity of a transcriptional regulatory region, said method comprising:
(a) providing at least a first and a second population of polynucleotide constructs,
(i) wherein each of said polynucleotide constructs in said first population comprises a first common transcriptional regulatory region operably linked to a candidate nucleotide sequence, and wherein each of said polynucleotide constructs in said first population further comprises a first common 5'UTR sequence;
(ii) wherein each of said polynucleotide constructs in said second population comprises a second common transcriptional regulatory region operably linked to a candidate nucleotide sequence, and wherein each of said polynucleotide constructs in said second population further comprises a second common 5'UTR sequence;
(iii) wherein said first and said second transcriptional regulatory regions are non-identical; and,
(iv) wherein said first and said second 5'UTR sequences are non-identical;
(b) introducing said populations of polynucleotide constructs into a population of host cells;
(c) screening for a modulation in the expression level of each candidate nucleotide sequence, and thereby identifying a nucleotide sequence that encodes a polypeptide that modulates the activity of said first or said second transcriptional regulatory region; and,
(d) determining if the identified nucleotide sequence encodes a polypeptide that modulates the activity of said first or said second transcriptional regulatory region.

21. The method of claim 20, wherein said determining if the identified nucleotide sequence encodes a polypeptide that modulates the activity of said first or said second transcriptional regulatory region comprises performing quantitative RT-PCR on mRNA isolated from said population of host cells.

22. The method of claim 21, wherein said quantitative RT-PCR further comprises using a primer specific to the identified nucleotide sequence and a primer specific to said first or said second 5'UTR sequence.

## Patentansprüche

1. Verfahren zur Identifizierung einer Nukleotidsequenz, welche für ein Polypeptid codiert, welches die Aktivität einer transkriptionell regulatorischen Region moduliert, wobei das Verfahren umfasst:
(a) Bereitstellen einer Population von Polynukleotidkonstrukten, wobei jedes der besagten Polynukleotidkonstrukte in besagter Population die transkriptionell regulatorische Region funktionell verknüpft mit einer Kandidatennukleotidsequenz umfasst;
(b) Einbringen besagter Population von Polynukleotidkonstrukten in eine Population von Wirtszellen und
(c) Screenen nach einer Modulation im Expressionsniveau von jeder Kandidatennukleotidsequenz und **dadurch** Identifizieren einer Nukleotidsequenz, welche für ein Polypeptid codiert, welches die Aktivität von besagter transkriptionell regulatorischer Region moduliert.

2. Verfahren gemäß Anspruch 1, wobei besagtes Screenen nach einer Modulation des Expressionsniveaus von jeder Kandidatennukleotidsequenz umfasst:
(a) Isolieren von mRNA aus besagter Population von Wirtszellen;
(b) Herstellen markierter Polynukleotide aus besagter isolierter mRNA und
(c) Hybridisieren besagter markierter Polynukleotide an einen komplementären DNA-Microarray.

3. Verfahren gemäß Anspruch 2, wobei besagtes Isolieren von mRNA aus besagter Population von Wirtszellen die bevorzugte Amplifikation von mRNA stammend aus besagter Population von Polynukleotidkonstrukten ermöglicht.

4. Verfahren gemäß Anspruch 1, wobei besagte Modulation einen Anstieg im Expressionsniveau einer Kandidatennukleotidsequenz umfasst.

5. Verfahren gemäß Anspruch 1, wobei besagte Modulation eine Abnahme im Expressionsniveau einer Kandidatennukleotidsequenz umfasst.

6. Verfahren gemäß Anspruch 1, wobei besagte transkriptionell regulatorische Region einen induzierbaren Promotor umfasst.

7. Verfahren gemäß Anspruch 6, wobei jedes der besagten Polynukleotidkonstrukte ferner ein Intron umfasst.

8. Verfahren gemäß Anspruch 7, wobei besagte transkriptionell regulatorische Region eine minimale Hintergrundaktivität aufweist.

9. Verfahren gemäß Anspruch 8, wobei besagtes Intron die Hintergrundexpression von besagter Kandidatennukleotidsequenz heraufsetzt.

10. Verfahren gemäß Anspruch 9, wobei besagtes Intron das Mais-Ubi1-Intron 1 ist.

11. Verfahren gemäß Anspruch 10, wobei besagter induzierbarer Promotor der Mais-Bronze-1-Promotor ist.

12. Verfahren gemäß Anspruch 1, wobei wenigstens eine der besagten Kandidatennukleotidsequenzen für ein Polypeptid codiert, welches die Aktivität von besagter transkriptionell regulatorischer Region direkt moduliert.

13. Verfahren gemäß Anspruch 1, wobei wenigstens eine der besagten Kandidatennukleotidsequenzen für ein Polypeptid codiert, welches die Aktivität von besagter transkriptionell regulatorischer Region indirekt moduliert.

14. Verfahren gemäß Anspruch 1, wobei besagte Wirtszellen Pflanzenzellen sind.

15. Verfahren gemäß Anspruch 14, wobei besagte Pflanzenzellen dikotyl sind.

16. Verfahren gemäß Anspruch 14, wobei besagte Pflanzenzellen monokotyl sind.

17. Verfahren gemäß Anspruch 14, wobei besagte Pflanzenzellen aus der Gruppe, bestehend aus Mais, Weizen, Sorghum, Reis, Gerste, Sojabohne, Alfalfa, Sonnenblume, *Brassica* und Tomate ausgewählt sind.

18. Verfahren gemäß Anspruch 1, wobei jede von besagten Kandidatennukleotidsequenzen aus einer Pflanzenzelle stammt.

19. Verfahren gemäß Anspruch 2, wobei besagte transkriptionell regulatorische Region die Expression einer Nukleotidsequenz, involviert in einen physiologischen Mechanismus von Interesse, reguliert, wobei besagter physiologischer Mechanismus von Interesse aus der Gruppe, bestehend aus einem Pathogenresistenz-Mechanismus, einer Gewebeentwicklung, einem Stoffwechselweg und einem Apoptoseweg ausgewählt ist.

20. Verfahren zur Identifizierung einer Nukleotidsequenz, welche für ein Polypeptid codiert, welches die Aktivität einer transkriptionell regulatorischen Region moduliert, wobei das Verfahren umfasst:
(a) Bereitstellen wenigstens einer ersten und einer zweiten Population von Polynukleotidkonstrukten,
(i) wobei jedes von besagten Polynukleotidkonstrukten in besagter erster Population eine erste allgemeine transkriptionell regulatorische Region umfasst, funktionell verknüpft mit einer Kandidatennukleotidsequenz, und wobei jedes von besagten Polynukleotidkonstrukten in besagter erster Population weiter eine erste allgemeine 5'-UTR-Sequenz umfasst;
(ii) wobei jedes von besagten Polynukleotidkonstrukten in besagter zweiter Population eine zweite allgemeine transkriptionell regulatorische Region umfasst, funktionell verknüpft mit einer Kandidatennukleotidsequenz, und wobei jedes von besagten Polynukleotidkonstrukten in besagter zweiter Population weiter eine zweite allgemeine 5'-UTR-Sequenz umfasst;
(iii) wobei besagte erste und besagte zweite transkriptionell regulatorische Region nicht identisch sind; und
(iv) wobei besagte erste und besagte zweite 5'-UTR-Sequenz nicht identisch sind;
(b) Einbringen besagter Population von Polynukleotidkonstrukten in eine Population von Wirtszellen;
(c) Screenen nach einer Modulation im Expressionsniveau von jeder Kandidatennukleotidsequenz und **dadurch** Identifizieren einer Nukleotidsequenz, welche für ein Polypeptid codiert, welches die Aktivität von besagter erster oder besagter zweiter transkriptionell regulatorischer Region moduliert und
(d) Bestimmen, ob die identifizierte Nukleotidsequenz für ein Polypeptid codiert, welches die Aktivität von besagter erster oder besagter zweiter transkriptionell regulatorischer Region moduliert.

21. Verfahren gemäß Anspruch 20, wobei besagtes Bestimmen, ob die identifizierte Nukleotidsequenz für ein Polypeptid codiert, welches die Aktivität von besagter erster oder besagter zweiter transkriptionell regulatorischer Region moduliert, die Durchführung einer quantitativen RT-PCR mit der mRNA, isoliert aus besagter Population von Wirtszellen umfasst.

22. Verfahren gemäß Anspruch 21, wobei besagte quantitative RT-PCR weiter die Verwendung eines Primers, spezifisch für die identifizierte Nukleotidsequenz und eines Primers spezifisch für die besagte erste oder besagte zweite 5'-UTR-Sequenz umfasst.

## Revendications

1. Procédé pour identifier une séquence de nucléotides qui code un polypeptide qui module l'activité d'une région régulatrice de la transcription, ledit procédé comprenant les étapes consistant à :
(a) fournir une population de constructions polynucléotidiques, chacune desdites constructions polynucléotidiques dans ladite population comprenant la région régulatrice de la transcription liée de manière opérationnelle à une séquence de nucléotides candidate ;
(b) introduire ladite population de constructions polynucléotidiques dans une population de cellules hôtes ; et
(c) rechercher une modulation du niveau d'expression de chaque séquence de nucléotides candidate, et ainsi identifier une séquence de nucléotides qui code un polypeptide qui module l'activité de ladite région de régulation de la transcription.

2. Procédé selon la revendication 1, dans lequel ladite étape consistant à rechercher une modulation du niveau d'expression de chaque séquence de nucléotides candidate comprend les étapes consistant à :
(a) isoler de l'ARNm à partir de ladite population de cellules hôtes ;
(b) préparer des polynucléotides marqués à partir dudit ARNm isolé ; et
(c) hybrider lesdits polynucléotides marqués à une micropuce d'ADN complémentaire.

3. Procédé selon la revendication 2, dans lequel ladite étape consistant à isoler de l'ARNm à partir de ladite population de cellules hôtes permet une amplification préférentielle de l'ARNm dérivé de ladite population de constructions polynucléotidiques.

4. Procédé selon la revendication 1, dans lequel ladite modulation comprend une augmentation du niveau d'expression d'une séquence de nucléotides candidate.

5. Procédé selon la revendication 1, dans lequel ladite modulation comprend une diminution du niveau d'expression d'une séquence de nucléotides candidate.

6. Procédé selon la revendication 1, dans lequel ladite région régulatrice de la transcription comprend un promoteur inductible.

7. Procédé selon la revendication 6, dans lequel chacune desdites constructions polynucléotidiques comprend en outre un intron.

8. Procédé selon la revendication 7, dans lequel ladite région régulatrice de la transcription a une activité de fond minime.

9. Procédé selon la revendication 8, dans lequel ledit intron augmente l'expression de fond de ladite séquence de nucléotides candidate.

10. Procédé selon la revendication 9, dans lequel ledit intron est l'intron Ubi1-intron1 du maïs.

11. Procédé selon la revendication 10, dans lequel ledit promoteur inductible est le promoteur bronze-1 du maïs.

12. Procédé selon la revendication 1, dans lequel au moins une desdites séquences de nucléotides candidates code un polypeptide qui module directement l'activité de ladite région régulatrice de la transcription.

13. Procédé selon la revendication 1, dans lequel au moins une desdites séquences de nucléotides candidates code un polypeptide qui module indirectement l'activité de ladite région de régulation de la transcription.

14. Procédé selon la revendication 1, dans lequel lesdites cellules hôtes sont des cellules végétales.

15. Procédé selon la revendication 14, dans lequel lesdites cellules végétales sont des cellules de dicotylédones.

16. Procédé selon la revendication 14, dans lequel lesdites cellules végétales sont des cellules de monocotylédones.

17. Procédé selon la revendication 14, dans lequel lesdites cellules végétales sont choisies dans le groupe consistant en du maïs, du blé, du sorgho, du riz, de l'orge, du soja, de l'alfalfa, du tournesol, des brassicacées et de la tomate.

18. Procédé selon la revendication 1, dans lequel chacune desdites séquences de nucléotides candidates est issue d'une cellule végétale.

19. Procédé selon la revendication 2, dans lequel ladite région régulatrice de la transcription régule l'expression d'une séquence de nucléotides impliquée dans une voie physiologique d'intérêt, ladite voie physiologique d'intérêt étant choisie dans le groupe consistant en une voie de résistance à un agent pathogène, une voie de développement tissulaire, une voie métabolique et une voie d'apoptotique.

20. Procédé pour identifier une séquence de nucléotides qui code un polypeptide qui module l'activité d'une région régulatrice de la transcription, ledit procédé comprenant les étapes consistant à :
(a) fournir au moins une première et une seconde population de constructions polynucléotidiques,
(i) chacune desdites constructions polynucléotidiques dans ladite première population comprenant une première région régulatrice de la transcription commune liée de manière opérationnelle à une séquence de nucléotides candidate, et chacune desdites constructions polynucléotidiques dans ladite première population comprenant en outre une première séquence 5'UTR commune ;
(ii) chacune desdites constructions polynucléotidiques dans ladite seconde population comprenant une seconde région régulatrice de la transcription commune liée de manière opérationnelle à une séquence de nucléotides candidate, chacune desdites constructions polynucléotidiques dans ladite seconde population comprenant en outre une seconde séquence 5'UTR commune ;
(iii) ladite première et ladite seconde régions régulatrices de la transcription étant différentes ; et,
(iv) ladite première et ladite seconde séquences 5'UTR étant différentes ;
(b) introduire lesdites populations de constructions polynucléotidiques dans une population de cellules hôtes ;
(c) rechercher une modulation du niveau d'expression de chaque séquence de nucléotides candidate et ainsi identifier une séquence de nucléotides qui code un polypeptide qui module l'activité de ladite première ou de ladite seconde région régulatrice de la transcription ; et
(d) déterminer si la séquence de nucléotides identifiée code un polypeptide qui module l'activité de ladite première ou de ladite seconde région régulatrice de la transcription.

21. Procédé selon la revendication 20, dans lequel ladite étape consistant à déterminer si la séquence de nucléotides identifiée code un polypeptide qui module l'activité de ladite première ou de ladite seconde région régulatrice de la transcription comprend l'étape consistant à réaliser une RT-PCR quantitative sur l'ARNm isolé à partir de ladite population de cellules hôtes.

22. Procédé selon la revendication 21, dans lequel ladite RT-PCR quantitative comprend en outre l'utilisation d'une amorce spécifique de la séquence de nucléotides identifiée et une amorce spécifique de ladite première ou de ladite seconde séquence 5'UTR.
